# EUROPÄISCHE PATENTSCHRIFT

(11) **EP 1 773 178 B1**
(45) Veröffentlichungstag und Bekanntmachung des Hinweises auf die Patenterteilung: **11.03.2009**
(21) Anmeldenummer: 04741143.4
(22) Anmeldetag: 19.07.2004
(51) Int. Cl.: A61B 1/313, A61B 1/05, A61B 5/00

(54) **VIDEOENDOSKOPIEVORRICHTUNG**
VIDEO ENDOSCOPY DEVICE
DISPOSITIF DE VIDEOENDOSCOPIE

(43) Veröffentlichungstag der Anmeldung: 18.04.2007
(73) Patentinhaber: Fraunhofer-Gesellschaft zur Förderung der angewandten Forschung e.V., 80686 München (DE)
(72) Erfinder: KRISCH, Ingo, 44799 Bochum (DE); BROCKHERDE, Werner, 47259 Duisburg (DE); HOSTICKA, Bedrich, 45478 Mühlheim (DE)
(74) Vertreter: Schoppe, Fritz
(86) Internationale Anmeldenummer: PCT/EP2004/008058
(87) Internationale Veröffentlichungsnummer: WO 2006/007865

(56) Entgegenhaltungen:
- WO-A-03/013624
- US-B1- 6 178 346

## Beschreibung

Die vorliegende Erfindung bezieht sich auf eine Videoendoskopievorrichtung, wie z.B. solche zur Darstellung der Innenwände des kardiovaskulären Systems bzw. solche, die für eine Verwendung im kardiovaskulären System geeignet sind.

Obwohl seit einigen Jahren die Forderung nach einer extrem miniaturisierten, Endoskopie-geeigneten hoch-auflösenden, und insbesondere "blutdurchdringenden" Kamera mit hoher Priorität seitens der Medizin gestellt wird, bedarf es zur Realisierung erheblicher technologischer Entwicklungsschritte, die bis vor kurzem noch in den Bereich der Utopie fielen. Bisher ist es weltweit noch nicht gelungen, ein Diagnosegerät zu entwickeln, das durch das Blut hindurch die Gefäßwände des kardiovaskulären Systems mit einer ausreichenden Auflösung darstellen kann. Seitdem Bozzini 1806 das erste Endoskop entwickelt hat, ist die optische Technik stark vorangetrieben worden und wurde auf vielfältige Anwendungen für die Inspektion mannigfaltiger Körperöffnungen und Organe spezialisiert. Dabei wird die klassische Fiberendoskopie immer mehr durch die moderne Videoendoskopie ersetzt, da diese erheblich bessere Bildauflösung und -qualität garantiert.

Beide Technologien versagen jedoch beim Einsatz in Blutgefäßen aufgrund der Lichtstreuung an den Hämoglobinmolekülen, ähnlich wie die Sicht im Nebel abhängig von der Tropfendichte und -größe stark eingeschränkt ist. Das Medium Blut verhält sich eigentlich optisch opak aufgrund der Mie-Streuung an den Erythrozyten und/oder aufgrund der starken Absorption der Wassermoleküle. Blut wird für die Strahlung im nahen Infrarotbereich (NIR) im Bereich zwischen 1,5 und 1,8 µm, sowie im Bereich zwischen 2,1 und 2,3 µm hinreichend transparent, so dass der Einsatz einer miniaturisierten NIR-Kamera Einblick in das bislang nur mit schwachen Umrissen erfassbare vaskuläre System liefert.

Bei der herkömmlichen Realisierung zur Darstellung der kardiovaskulären Systems werden derzeit im Wesentlichen vier verschiedene Methoden verwendet.

Bei den Ultraschallverfahren kann mittels eines Verfahrens der Doppler-Technik, das sich Duplex-Verfahren nennt, sowohl die Herzbewegung verfolgt werden, als auch das Arterien- und Venensystem dargestellt werden. Da dieses Verfahren jedoch eigentlich zur Flussmessung dient, kann die Bildauflösung nicht den Ansprüchen eines Herzchirurgen gerecht werden.

Bei der Computertomographie wird nach Injektion eines Radiopharmakons mit Hilfe der Emissionscomputertomographie (ECT = emission computer tomography) die Aktivitätsverteilung verschiedener Körperschichten zweidimensional erfasst. Zwar erlaubt die Anreicherung von TC oder I im Gefäßsystem eine Darstellung der Arterien und Venen, jedoch verursachen starke Inhomogenitäten und Dissymmetrien große Artefakte (Fehldarstellungen), wie bei der Herzuntersuchung durch Lungen- oder Mammaabsorption. Infolge der Artefakte reicht die Bildqualität dieses bildgebenden Verfahrens für die Herzchirurgie nicht aus. Zudem versagt das vorgenannte Verfahren auch bei der Darstellung von bewegten Bildern.

Bei der Magnetresonanztomographie erlaubt die (EKGgetriggerte) Phasenkontrast-Angiographie eine grobe Darstellung des Gefäßsystems, jedoch nicht in Echtzeit und gehört zur klinischen Routine.

Im Moment wird die Technik der modifizierten Ballonkatheter noch diskutiert und erprobt, jedoch ohne Perspektiven auf einen durchschlagenden Erfolg: Bei diesem Verfahren wird ein Ballon-Katheter in das kardiovaskuläre System eingeführt und vom behandelnden Arzt durch die Venen zum Untersuchungsort geschoben, bevor der Ballon-Katheter dort mit Ringerlösung aufgeblasen wird. Die transparente Hülle des Ballons drückt dabei direkt gegen die Gefäßwand, so dass ein optisches System, das in den Ballon integriert ist, die Struktur der Wand abbilden kann. Die Nachteile dieser Methode liegen einerseits in einem kompletten Gefäßverschluss, andererseits in der hohen Druckbelastung auf die Gefäße.

Auf der technischen Seite wird die klassische Fieberendoskopie im Hinblick auf schmale Durchmesser optimiert, indem Quarzfasern mit einem Durchmesser von 2,8 µm als lichtleitende Faser eingesetzt werden. Obwohl auf diese Weise sehr kleine Pixel realisiert werden können, besitzt das Verfahren wegen der hohen Lichtverluste im sichtbaren Bereich und der geringen numerischen Apertur einige Nachteile. Trotz starker Beleuchtung des Untersuchungsortes liefert dieses Verfahren nur Bilder mit geringer Helligkeit, zumal die Transmission im infraroten Bereich sich gegenüber dem sichtbaren Bereich verschlechtert.

In der US-6,178,346 wird ein Infrarot-Fieberendoskopie-Verfahren beschrieben, das in den USA unter dem Warenzeichen Transblood Vision registriert ist. Blut ist eigentlich opak aufgrund Mie-Streuung an den Enthrozyten und/oder aufgrund der starken Absorption der Wassermoleküle. Das in der US-6,178,346 vorgeschlagene Verfahren umgeht jedoch die Probleme durch besondere Wahl der Infrarotwellenlänge. Bei dem Verfahren wird von einer Laserdiode erzeugte Strahlung über einen Strahlteiler in eine lichtleitende Faser des Endoskops eingekoppelt, womit die Untersuchungsstelle beleuchtet wird. Das von der Untersuchungsstelle reflektierte Licht wiederum wird über das proximale Ende des Katheters über den Strahlteiler an einen externen Kamerasensor weitergeleitet. Ein Nachteil an der dort vorgeschlagenen Vorgehensweise ist die erhebliche Dämpfung des die bildgebenden Informationen enthaltenen optischen Signals und damit die Begrenzung der erreichbaren Objekthelligkeit.

Die WO 03/013624 A2 beschreibt eine intravaskuläre Abbildungsvorrichtung mit einem Katheter, bei dem der Bildsensor an dem distalen Ende angeordnet ist ebenso wie Beleuchtungsquellen zur Beleuchtung der zu beobachtenden Region vor dem distalen Ende, einer Optik zur Abbildung des zu betrachtenden Bereiches auf dem Bildsensor und ein Prisma zum Ablenken der bildseitigen Strahlen zwischen der Optik und dem Bildsensor, so dass der Bildsensor nicht-senkrecht zu einer Längsachse des Katheters angeordnet ist. I/O-pads sind neben der eigentlichen bildaufnehmenden Fläche angeordnet, so dass der Bildsensor insgesamt länglich ist. Die kleine Breite der Bildfläche mache es möglich, den Durchmesser des Katheters zu reduzieren.

Es ist Aufgabe der Erfindung, eine Videoendoskopvorrichtung zu schaffen, die eine verbesserte Bildqualität ermöglicht, wie z.B. bei prä-, intra- und postoperativen Beobachtungen an bzw. in Organen und Gefäßen in realer, das heißt, bluterfüllter Umgebung, und das bei geringerem Aufwand für Kontaktierung bzw. Verkabelung.

Diese Aufgabe wird durch eine Videoendoskopievorrichtung gemäß Anspruch 1 gelöst.

Eine erfindungsgemäße Videoendoskopievorrichtung umfasst eine (Bild-)Sensorvorrichtung und einen Katheter zum Leiten von Strahlung an ein distales Ende des Katheters und Ausgeben derselben an dem distalen Ende des Katheters, und zum Empfangen reflektierter Strahlung an dem distalen Ende und Abbilden derselben auf die Sensorvorrichtung. Die Sensorvorrichtung ist im Katheter in der Nähe des distalen Endes des Katheters angeordnet und ausgebildet, um die reflektierte Strahlung in ein elektrisches Signal umzuwandeln. Der Katheter ist ausgebildet, um das elektrische Signal zu einem proximalen Ende des Katheters zu leiten.

Der Kerngedanke, der der Erfindung zugrunde liegt, besteht darin, eine Beleuchtung eines zu untersuchenden Objekts durch eine beispielsweise von einer lichtleitenden Faser übertragenen Strahlung durchzuführen, während die Erfassung der Rückstreustrahlung durch einen in der Katheterspitze angeordneten Sensor vorgenommen wird, um das Bild des Objekts in ein elektrisches Signal umzuformen, was über beispielsweise eine Kabel- oder Leitungsverbindung einer externen Bildverarbeitungsvorrichtung zugeführt werden kann. Dadurch kann auf eine Bildübertragung mittels Glasfaserkabel verzichtet werden, und damit können die Beeinträchtigungen der Bildqualität durch die optische Dämpfung des Signals, insbesondere auf dem Rückweg von der Katheterspitze zur externen Einheit vermieden werden.

Bevorzugte Ausführungsbeispiele der vorliegenden Erfindung werden nachfolgend bezugnehmend auf die beiliegenden Zeichnungen näher erläutert. Es zeigen:
- Fig. 1: ein schematisches Blockdiagramm einer kardiovasku- lären Videoendoskopvorrichtung;
- Fig. 2: eine schematische Querschnittsansicht einer dista- len Katheterspitze bzw. eines Katheterkopfs gemäß einem Ausführungsbeispiel der vorliegenden Erfin- dung; und
- Fig. 3: ein schematisches Raumbild eines Bildsensors in der distalen Katheterspitze von Fig. 2.

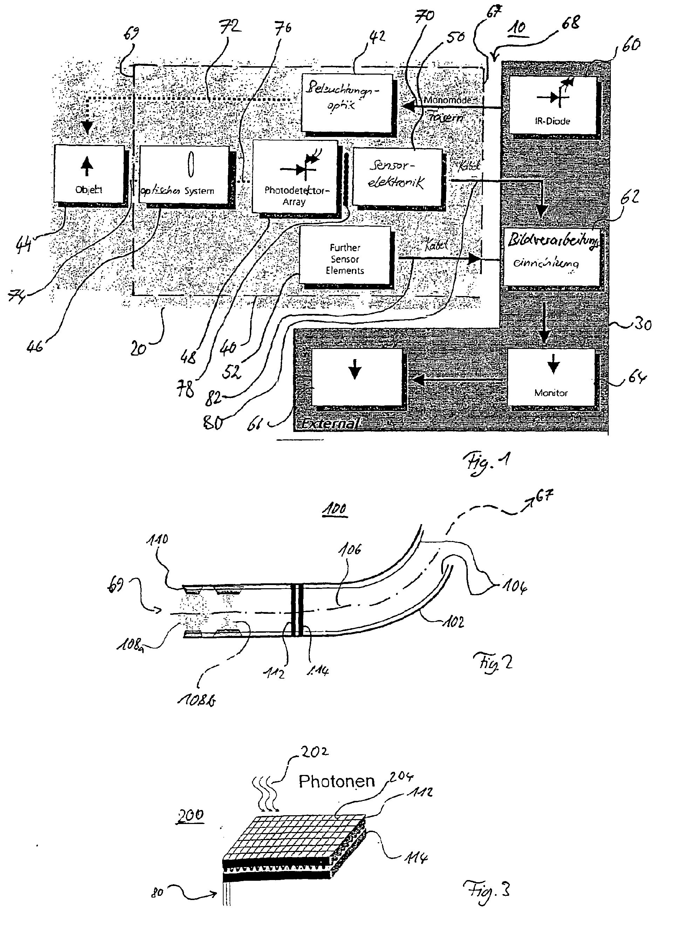

Fig. 1 zeigt eine Videoendoskopievorrichtung die gemäß einem Ausführungsbeispiel der vorliegenden Erfindung ausgebildet sein kann. Die Videoendoskopievorrichtung, die allgemein mit 10 angezeigt ist, gliedert sich im Wesentlichen in zwei Teile, nämlich einen beweglichen Teil 20 und einen externen, statischen Teil 30.

Der bewegliche Teil 20 bildet eine bewegliche Katheteranordnung. Er weist insbesondere einen Katheter 40 auf, in welchem eine Optik 42 zur Beleuchtung eines zu untersuchenden Objektes 44, wie z.B. die Gefäßwand einer Blutbahn, ein optisches System 46 zur Abbildung des beleuchteten Objekts 44 auf ein ebenfalls in dem Katheter 40 angeordnetes Photodetektorarray als Sensor 48, eine Vorverarbeitungsschaltung bzw. Sensorelektronik 50 und wahlweise weitere SensorElemente 52 enthalten sind. Die Sensorelektronik 50 besteht vorzugsweise aus einer Sensoransteuerung, einer Ausleseschaltung und einer Bildvorverarbeitungseinheit.

Der statische Teil 30 bildet im Wesentlichen die externe Apparatur der Videoendoskopievorrichtung 10. Er umfasst eine Strahlungsquelle 60, eine Bild- und Signalverarbeitungseinrichtung 62, eine Anzeigeeinheit bzw. einen Monitor 64 und einen Speicher 66.

Der Katheter 40 ist an einem proximalen Ende 67 desselben mit der externen Apparatur 30 koppelbar, wie z.B. über eine lösbare oder feste Steckverbindung. Die Schnittstelle zwischen Katheter 40 und externer Apparatur 30 wird in Fig. 1 mit 68 angezeigt. Mit einem distalen Ende 69 bzw. einer Katheterspitze ist der Katheter 40 dem Objekt 44 bzw. dem auszuleuchtenden Untersuchungsbereich zuwendbar.

Ein Strahlungsleiter 70, wie z.B. eine Mehrzahl von Monomodefasern, wie es später bezugnehmend auf Fig. 2 exemplarisch beschrieben wird, verläuft in dem Katheter 40 zwischen dem proximalen Ende 67 und der Optik 42, um die von der Strahlungsquelle 60 erzeugte Strahlung zur Optik 42 zu leiten, die diese Strahlung homogen auf dem Objekt 44 verteilt, wie es durch eine Strichlinie 72 angedeutet ist. Die Optik 42 muss nicht eine eigens vorgesehene Optik sein, sondern kann ferner durch das Austrittsende des Strahlungsleiters 70 selbst gebildet sein. Eine gestrichelte Linie 74 soll in Fig. 1 einen weiteren Strahlungsweg zwischen dem Objekt 44 und dem optischen System 46 darstellen, nämlich denjenigen, auf welchem das von dem Objekt reflektierte Licht in das optische System 46 gelangt. Ein dritter Strahlungsweg 76 befindet sich zwischen dem optischen System 46 und dem Sensor 48. Auf diesem Strahlungsweg 76 bildet das optische System 46 das Objekt 44 auf das Pixelarray 48 oder, genauer ausgedrückt, auf die lichtempfindliche Fläche des Pixelarrays 48 ab, das aus einem Array von Pixeln besteht und aus der Abbildung mit einer bestimmten Wiederholfrequenz Pixelmesswerte für alle Pixel und damit Bilddarstellungen erzeugt.

Zwischen dem Sensor 48 und der Elektronik 50 befindet sich ein elektrisches Verbindungssystem 78 zur elektrischen Verbindung derselben bzw. für eine Weiterleitung der Pixelmesswerte an die nachfolgende Schaltung 50. Ein elek-trischer Leiter 80, wie z.B. ein oder eine Mehrzahl von Kabeln, verläuft zwischen der Schaltung 50 und dem proximalen Ende 67 des Katheters 40, um aus den Pixelmesswerten durch die Schaltung 50 erhaltene vorverarbeitete Bilddaten im angekoppelten Zustand des Katheters 40 über die Schnittstelle 68 an die Bildverarbeitung 62 weiterzuleiten. Die Daten werden somit über eine definierte Schnittstelle an eine zu dem Katheter 40 externe Hardware, hier die Bildverarbeitung 62, übergeben. Ein weiterer elektrischer Leiter 82, wie z.B. ein oder eine Mehrzahl von Kabeln, ist zwischen den wahlweisen weiteren Sensorelementen 52 und der Bild- bzw. Signalverarbeitung 62 angeordnet bzw. verläuft hierzwischen, um Messdaten der Sensorelemente 52 an die Verarbeitungseinrichtung 62 weiterzuleiten.

Wie später bezugnehmend auf Fig. 2 deutlicher werden wird, sind die Optik 42, das optische System 46, das Photodetektorarray 48 und die Schaltung 50 sowie die weiteren Sensorelemente 52 in der Nähe des distalen Endes des Katheters 40 angeordnet und bilden somit den Katheterkopf bzw. eine Katheterspitze des Katheters 40.

Innerhalb der externen Apparatur 30 ist die Bildverarbeitung 62 mit der Schnittstelle 68 zum Ankoppeln des proximalen Endes 67 des Katheters 40 verbunden, um im angekoppelten Zustand des Katheters 40 die vorverarbeiteten Daten über das Kabel 80 von der Vorverarbeitungseinrichtung 50 und die Sensormessdaten von den wahlweisen Sensoren 52 über das Kabel 82 zu erhalten. Ein Ausgang der Verarbeitungseinrichtung 62 ist mit dem Eingang des Monitors 64 verbunden, um das im Photodetektorarray 48 erhaltene Bild des Objekts 44 dem Benutzer der Vorrichtung 10 anzeigen zu können sowie gegebenenfalls laufende Messergebnisse der zusätzlichen Sensoren 52. Ferner ist der Ausgang der Verarbeitungseinrichtung 62 mit dem Speicher 66 verbunden, um die von der Vorverarbeitungseinrichtung 50 und den Sensorelementen 52 erhaltenen Daten archivieren zu können, wie z.B. für eine spätere Auswertung der Daten.

Mit der Schnittstelle 68 ist - diesmal jedoch auf eine optische Art und Weise - ferner die Infrarotdiode 60 verbunden, um Licht über die Schnittstelle 68 in den Strahlungsleiter 70 des Katheters 40 einkoppeln zu können, sobald dieser an die Apparatur 30 angekoppelt ist.

Nachdem im vorhergehenden der Aufbau der Vorrichtung 10 beschrieben worden ist, wird um folgenden kurz ihre Funktionsweise beschrieben.

Zwecks Beleuchtung des Untersuchungsortes 44 wird Strahlung extern von der Lichtquelle 60, bei der es sich im folgenden exemplarisch um eine Infrarot-Diode handeln soll, erzeugt. Diese Bestrahlung wird dann via dem Lichtleiter 70 bzw. den Monomodefasern im Fall des Ausführungsbeispiels von Fig. 2 durch den Katheter 40 transportiert und über die Optik 42 homogen auf die auszuleuchtende Fläche bzw. das Objekt 44 verteilt. Die ausgeleuchtete Szenerie 44 streut das Licht zurück in das optische System 46. Dieses optische System 46 bildet die ausgeleuchtete Szene mit einem gewissen Tiefenschärfebereich auf das Photodetektorarray 48 ab, wo das Bild mit einer gewissen Auflösung, die von dem Pixelabstand des Photodetektorarrays 48 abhängt, in ein Array von Pixelmesswerten ungewandelt wird. Die Pixelmesswerte werden wiederum über das Verbindungssystem 78 an die Sensorelektronik 50 weitergeleitet, die zunächst die Daten ausliest und anschließend eine gewisse Vorverarbeitung der bis dahin beispielsweise noch analogen Pixelmesswerte vornimmt, wie z.B. eine reine Digitalisierung, eine Dynamikanpassung oder dergleichen. Damit das Photodetektorarray 48 und die Vorverarbeitungseinrichtung 50 ihre Aufgaben erfüllen können, werden dieselben beispielsweise über die elektrische Leiterverbindung 80 mit Energie versorgt. Die vorverarbeiteten Daten gelangen zur Bildverarbeitungseinrichtung 62, wo die Daten derart aufbereitet werden, dass sie als Videosignal vorliegen und durch den Monitor 64 anzeigbar sind. Ein Arzt, der die Vorrichtung 10 verwendet, kann nun, nachdem er den Katheter 40 in das Arterien- und Venensystem eingeführt hat, unter Beobachtung des Monitors 64 durch Drehen und Biegen des Katheters 40 das distale Ende 69 bzw. den Bildausschnitt des optischen Systems 46 an den gewünschten Untersuchungsort 44 navigieren.

Über die weiteren Sensorelemente 52 ist es dem Arzt möglich, weitere Informationen über den Untersuchungsort 44 zu gewinnen, wie z.B. eine Blutflussmessung durch einen Flussmesser, eine Temperaturmessung durch einen Temperatursensor oder dergleichen. Diese Messwerte können dann für eine weitere Diagnostik und Kontrolle verwendet werden. Es wird darauf hingewiesen, dass es möglich ist, dass der Arzt gegebenenfalls über eine in Fig. 1 nicht gezeigte Eingabeeinrichtung, wie z.B. eine Tastatur, Einstellungen an der Vorverarbeitungseinrichtung 70 oder dem Photodetektorarray 48 vornimmt, wie z.B. eine Veränderung der Auflösung bei gleichzeitiger entsprechender Veränderung der Bildwiederholrate oder dergleichen.

Nachdem nun im vorhergehenden bezugnehmend auf Fig. 1 eher allgemein ein Ausführungsbeispiel der vorliegenden Erfindung beschrieben worden ist, wird im folgenden ein Ausführungsbeispiel für eine Katheterspitze detaillierter beschrieben. Die in Fig. 2 gezeigte Katheterspitze ist allgemein mit 100 angezeigt. Wieder kurz bezugnehmend auf Fig. 1 ist die Katheterspitze 100 von Fig. 2 am distalen Ende 69 angeordnet. Der in Fig. 2 nicht gezeigte Teil des Katheters 40 führt weiter bis zum proximalen Ende 67 des Katheters wie es durch einen gestrichelten Teil angedeutet ist, der gebogen ist, um die Biegsamkeit des Katheters anzudeuten.

Die Katheterspitze 100 von Fig. 2 ist schematisch im Querschnitt dargestellt. Zu sehen ist die schlauchförmige und flexible Ummantelung 102 des Katheters. Sie bildet den Außenmantel des Katheters. Im Inneren des Katheters entlang der Ummantelung 102 verlaufen vom proximalen Ende 67 bis zum distalen Ende 69 Monomodefasern 104. In einem Querschnitt quer zur Längsachse 106 des Katheters sind sie ringförmig um die Längsachse 106 herum entlang der Innenwand der Ummantelung 102 angeordnet. Die Monomodefasern 104 bilden somit den Strahlungsleiter 70 von Fig. 1 und transportieren das Licht der Infrarotdiode 60 zum distalen Ende 69.

Achssymmetrisch zur Längsachse 106 sind zum Abschluss des Katheters am distalen Ende 69 Linsen 108a und 108b angeordnet, welche das optische System 46 des Katheters bilden. Sie sind über ringförmige Halterungen 110 an der Innenseite der Ummantelung 102 angebracht. Durch diese Halterungen 110 verlaufen auch die Monomodefasern 104, um ihr Licht am distalen Ende 69 ausgeben zu können. Gegebenenfalls sind in den Halterungen 110 pro Monomodefaser 104 Elemente zur Strahlaufweitung vorgesehen. Alternativ bilden die Abschlussenden der Monomodefasern 104 am Austrittspunkt an den Halterungen 110 oder kurz danach die Optik 42 aus Fig. 1. In einem bestimmten Abstand hinter den Linsen 108a-108b, d.h. in Richtung des proximalen Endes 67, ist ein Verbund aus einem Photodetektorarray 112 und einem Halbleiterchip 114 quer zur Längsachse 106 angeordnet, von denen letzterer die Sensorelektronik 50 bildet. Der Verbund 112, 114 ist vorzugsweise ebenfalls achssymmetrisch zur Längsachse 106 im Katheter angeordnet und an den Innenwänden der Ummantelung 102 befestigt, und zwar derart, dass die an der Innenwand der Ummantelung 102 vom proximalen 67 zum distalen Ende 69 verlaufenden Monomodefasern 104 den Verbund 112, 114 passieren können. In dem sich an den Verbund 112, 114 zum proximalen Ende 67 hin anschließenden Teil des Katheters verlaufen die lediglich in Fig. 1 gezeigten und in Fig. 2 zur Übersichtlichkeit weggelassenen Kabel zur Energieversorgung des Verbunds 112, 114 und zur Weiterleitung der Daten aus dem Verbund 112, 114 zur Bildverarbeitungseinrichtung 62 usw.

Die Katheterspitze von Fig. 2 wäre ohne weiteres dazu geeignet, bei der Vorrichtung nach Fig. 1 eingesetzt zu werden. In Fig. 2 fehlte dann lediglich neben der Darstellung der Kabel noch die Darstellung der weiteren Sensoren 52. Diese könnten beispielsweise an der Außenhaut der Ummantelung 102 vorgesehen sein, oder an dem distalen Ende 69 an der freigelegten Seite der Halterung 110.

Bezugnehmend auf Fig. 3 wird ein Ausführungsbeispiel für den Verbund 112, 114 aus Fig. 2 beschrieben. Fig. 3 zeigt den Verbund allgemein mit einem Bezugszeichen 200 an. Dargestellt ist der Verbund 200 in Fig. 3 in einer Raumdarstellung aus einer Perspektive, bei der diejenige Seite des Verbunds 200 sichtbar ist, die dem distalen Ende 69 bzw. der Optik 108a-108b (Fig. 2) zugewandt ist, und auf die von dem Objekt rückgestreute Photonen auf den Verbund 200 treffen, wie es durch Pfeile 202 angedeutet ist. Der Verbund 200 besteht aus dem Photodetektorarray 112 und dem Halbleiterchip 114. Das Photodetektorarray 112 ist in einem Halbleitersubstrat gebildet, wie z.B. in einem III-V-Halbleiter, wie z.B. in einem InGaAs-Halbleiter. In dem Halbleitersubstrat sind Photodioden gebildet, derart, dass die Photodioden ein Array von Pixeln ergeben, wie es in Fig. 3 durch die Arrayeinteilung 204 angedeutet ist. Das Halbleitersubstrat, in dem das Photodiodenarray 112 gebildet ist, ist jedoch mit einer Hauptseite die Strahlung 202 bzw. dem distalen Ende 69 zugewandt, die der Hauptseite dieses Halbleitersubstrates gegenüberliegt, in welcher das Photodiodenarray tatsächlich in diesem Halbleitersubstrat gebildet ist. Die Photonen 202, die nach Rückstreuung am Objekt 44 eintreffen, treten also zunächst durch die Hauptseite 204 des Halbleitersubstrates des Photodiodenarrays 112 in das Halbleitersubstrat ein, um nach Hindurchtreten auf das Photodiodenarray in der der Hauptseite 204 gegenüber liegenden Hauptseite des Halbleitersubstrats bzw. auf die Raumladungszonen zu treffen und dort via Diffusion- und/oder Driftstrom in Pixelmesssignale umgewandelt zu werden.

Mit Hilfe von Flip-Chip-Bonding als einem Beispiel einer Methode der Aufbau- und Verbindungstechnik (AVT) ist das so gebildete Photodiodenarray 112 auf einen Halbleiterchip aufgesetzt, wie z.B. einem CMOS-Chip 114, in welchem die Vorverarbeitungseinrichtung 50 integriert ist. Das Photodiodenarray 112 und der Chip 114 sind dabei derart miteinander verbunden, dass die Hauptseite des Halbleitersubstrates, in welchem das Photodiodenarray 112 gebildet ist, dem Halbleiterchip 114 mit derjenigen Hauptseite zugewandt ist, in welcher das Photodiodenarray 112 gebildet ist, d.h. mit der der Hauptseite 204 abgewandten Seite, bzw. mit derjenigen Hauptseite, die dem distalen Ende abgewandt ist. Der Halbleiterchip 114 wiederum ist derart mit dem Photodiodenarray 112 verbunden, dass er demselben mit derjenigen Hauptseite des Chips zugewandt ist, in der die Schaltung integriert ist, die die Ansteuer-, Auslese- und Vorverarbeitungselektronik 50 bildet. In Fig. 3 sind auch noch die Kabel 80 aus Fig. 1 gezeigt, die für die Energieversorgung des Verbunds 200 zuständig sind bzw. für die Weiterleitung der verarbeiteten Daten von dem Chip 114 zu der Bildverarbeitungseinrichtung 62 oder von Steuersignalen umgekehrt von der Verarbeitungseinrichtung 62 zu dem Chip 114 bzw. der darauf integrierten Schaltung.

Eine spezielle Ausgestaltung eines Videoendoskops gemäß allen vorhergehenden Ausführungsbeispielen von Fig. 1-3, also eines Videoendoskops, das den Aufbau von Fig. 1, die Katheterspitze gemäß Fig. 2 und den Photodetektorarray/Vorverarbeitungschip-Verbund gemäß Fig. 3 aufweist, mit Anpassung zur kardiovaskulären Untersuchung könnte als externe Strahlungsquelle eine Infrarotdiode, als Strahlungsleiter 70 mehrere Monomodefasern, welche die Strahlung an den Untersuchungsort 44 heranführen, als Zuleitungen 80 und 82 Kabel zur Energieversorgung des Pixelarray/Vorverarbeitungsverbunds und zur Datenauslese, als einen Bildsensor 48 ein Detektorarray 112 auf einem III-V-Halbleiter, der mit Hilfe von beispielsweise Flip-Chip-Bonding auf einer Auslese-, Vorverarbeitungs- und Ansteuerelektronik, die auf einem darunter liegenden CMOS-Chip 114 integriert ist, aufgesetzt ist, als Optik 46 ein Linsensystem zur optischen Abbildung mit nötiger Schärfentiefe und ausreichendem Sichtfeld mit gegebenenfalls Autofocus, als Verarbeitungseinrichtung einen Prozessor 62 zur Bildverarbeitung und als Monitor 64 beispielsweise ein TFT-Monitor, von denen die beiden letztgenannten beispielsweise in einem externen Modul 30 eingebaut werden und über Kabel 80 den Bildsensor 48 ansteuern, sowie als eventuelle weitere Hilfsapparaturen solche zur Steuerung bzw. Drehung des distalen Katheterendes, also eine Navigationshilfe, sowie evtl. mehrere Sensoren 52 zwecks weiterer Diagnostik und Kontrolle, wie z.B. für den Blutfluss, die Temperatur, usw. aufweisen. Ein solcherart miniaturisierter Katheter, der den Bildsensor 48, die Optik 46, die Monomodefasern für die Beleuchtung und die Kabel durch das Arterien- bzw. Venensystem an den Untersuchungsort heranführt, sollte biokompatibel und stabil verkapselt sein. Dies gilt insbesondere für die Ummantelung 102, d.h. sie sollte biokompatibel und steril sein.

Ein solcherart gebildetes kardiovaskuläres Videoendoskop vereinfacht die Planung, die Durchführung und die anschließende Überwachung medizinischer Eingriffe im Gefäßsystem des Menschen wesentlich. Defekte des kardiovaskulären Systems können hiermit direkt in Blut erfüllter Umgebung bewertet werden. Dank der verminderten Eingriffszeit resultiert daraus eine insgesamt patientenschonendere Behandlung. Nach Etablierung des Verfahrens können die Behandlungskosten drastisch gesenkt werden. Im Vergleich zu bisherigen Diagnosesystemen leistet ein solcherart gebildetes Endoskop eine deutlich höhere Bildauflösung. Mit den Verfahren moderner Bildverarbeitungen, wie z.B. der Mustererkennung, die beispielsweise in der Verarbeitungseinrichtung 62 vorgenommen wird, oder aber einer anderen Prozessoreinheit, die auf den Speicher 66 Zugriff hat, könnte eine vom Arzt gewünschte Information sofort aus den durch den Katheter gewonnenen Daten hergeleitet werden.

Wie es im vorhergehenden schon beschrieben worden ist, sind die Sensorelemente 52 nicht unbedingt erforderlich. Beispiele für solche das distale Ende des Endoskops in der Katheterspitze nach den Bedürfnissen des Anwenders erweiternden sensorischen Elementen umfassen einen Flusssensor, einen Temperatursensor, chemische Sensoren oder dergleichen.

Im Vergleich zu dem in der Beschreibungseinleitung erwähnten Verfahren aus der US 6,178,346 ist bei einem Videoendoskop gemäß der vorliegenden Erfindung die Kameravorrichtung bzw. der Bildsensor in-situ angebracht. Insoweit kann auch auf die Bildübertragung mittels Glasfaserkabel verzichtet werden. Da solche Kabel eine niedrigere Apertur besitzen und außerdem das optische Signal dämpfen, ist die Bildqualität bei dem herkömmlichen Verfahren vergleichsweise schlecht. Die im vorhergehenden beschriebenen Ausführungsbeispiele versprechen hingegen eine wesentlich bessere Bildqualität.

Eine Endoskopievorrichtung gemäß der vorhergehenden Ausführungsbeispiele, die zur kardiovaskulären Untersuchung geeignet sein soll, sollte im Gegensatz zu herkömmlichen Videoendoskopen, die den sichtbaren Wellenlängenbereich von 400-700 nm nutzen, bei einer Wellenlänge von 2,1 µm arbeiten. Sowohl eigene theoretische Berechnungen als auch experimentelle Untersuchungen bestätigen, dass Blut bei dieser Wellenlänge hinreichend transparent ist. Die Auswahl der Wellenlänge resultiert aus einem Kompromiss: bei niedrigen Wellenlängen ist die Lichtstreuung an den Partikeln zu groß, bei höheren Wellenlängen ist die Absorption durch den hohen Wasseranteil zu hoch. Die erreichbare Sichtweite beträgt im Blut ca. 12 mm bei dieser Wellenlänge. Denkbar wäre auch ein Videoendoskop, das bei einer Wellenlänge von 1,7 µm arbeitet. Die erreichbare Sichtweite läge in diesem Fall bei 8 mm. Andere Wellenlängenbereiche, wie z. B. zwischen 1,5µm und 1,8µm oder zwischen 2,1µm und 2,3µm können aber ebenfalls ausreichend sein.

Noch einmal in anderen Worten ausgedrückt umfasst der im vorhergehenden bezugnehmend auf Fig. 1 vorgeschlagene Gesamtaufbau der Videoendoskopievorrichtung eine Strahlungsquelle, ein Kabel mit Zuleitungen und Monomodefasern, um die Beleuchtung vor Ort zu ermöglichen, einen Bildsensor mit Elektronik, eine Optik, einen Prozessor, einen Monitor und evtl. weitere Steuerungsgeräte und Sensoren. Gemäß einer bestimmten Ausgestaltung könnte das Videoendoskop einen miniaturisierten, verkapselten Katheterkopf umfassen, wie er beispielsweise in Fig. 2 gezeigt ist. Es könnte zusätzlich zu dem in Fig. 2 gezeigten Bildsensorarray, der Optik, der Auslese- und Ansteuerungselektronik, den Schnittstellen und der Beleuchtungseinheit auch noch weitere Bildsensorarrays umfassen. Mit Hilfe der zusätzlichen Bildsensoren kann beispielsweise einerseits das Bildfeld vergrößert werden, indem diese beispielsweise seitlich in die distale Katheterspitze integriert werden, andererseits könnten die zusätzlichen Bildsensoren unterschiedliche Abbildungsverfahren verwenden, so dass über die jeweils anderen Abbildungsverfahren weitere bzw. zusätzliche Informationen gewonnen werden. Zu diesen Abbildungsverfahren gehört beispielsweise die laserinduzierte Fluoreszenz (LIF) oder das Streulichtverfahren. Für eine detailliertere Untersuchung könnte der zu untersuchende Bereich beispielsweise eingefärbt bzw. mit einem bestimmten Stoff angereichert werden, so dass sich bei Beleuchtung das Reflexionsverhalten lokal ändert. Auf diese Weise könnten unterschiedliche Oberflächenstrukturen besser diskriminiert werden.

Ferner ist es möglich, anstatt einer extern angeordneten Lichtquelle Lichtquellen direkt in das distale Ende des Kathederkopfes zu integrieren, wie z. B. Photodioden. Diese könnten dann im Ausführungsbeispiel von Fig. 2 beispielsweise an den Stellen angeordnet sein, die den Austrittsstellen der dortigen Lichtleiter 104 entsprechen. Anstelle der Lichtleiter 104 benötigte man hier lediglich elektrische Zuleitungen zur Versorgung der Photodioden bzw. Lichtquellen mit der notwendigen Leistung.

Wie bezugnehmend auf Fig. 3 beschrieben, könnte gemäß einer Ausgestaltung das Bildsensorarray auf der Basis eines III-V-Halbleiters, wie z.B. als InGaAs-Photodiodenarray gebildet sein und mit Hilfe von Flip-Chip-Bonding auf einem CMOS-Chip aufgesetzt werden. Der dann darunter liegende CMOS-Chip könnte die Auslese- Vorverarbeitungs- und Ansteuerungselektronik für den oberen Chip beinhalten. Auf dem darunter liegenden CMOS-Chip könnte auch die Schnittstellenelektronik integriert sein, mit der die akquirierten Bildsignale über das Kabel zum Prozessor in der externen Apparatur übertragen werden können. Die eigentliche Signal- und Bildverarbeitung läuft in dem externen Prozessor mit einer bedienerfreundlichen Schnittstelle ab, bevor die Bilder auf einem Monitor angezeigt werden.

Da der äußere Durchmesser durch die Gefäße begrenzt ist - die größeren Arterien bzw. Venen besitzen einen Durchmesser zwischen 6 und 14 mm - sollte der Aufbau des Katheters der obigen Ausführungsbeispiele bei Verwirklichung hinreichend miniaturisiert sein. Der minimale Photodiodenabstand bzw. - Pitch ist durch die beugungsbegrenzte Auflösung mit 7 µm vorgegeben, so dass ein Videoendoskop mit einem Durchmesser von 1,5 mm theoretisch eine Auflösung von 20.000 Bildpunkten (Pixeln) bieten könnte.

Die Optik bzw. das optische System sollte mindestens einen Sehbereich von 25 Grad ermöglichen. Das optische System sollte in einem Bildweitenbereich zwischen 5 und 12 mm autofocussieren. Der Linsendurchmesser sollte 3 mm nicht übersteigen. Die Bildrate des Bildsensors sollte mindestens 15 Bilder pro Sekunde betragen.

Ein Katheterkopf nach den Ausführungsbeispielen von Fig. 2 und 3 könnte von einem Arzt derart eingesetzt werden, dass der Katheter vom Arzt an die zu untersuchende Stelle gebracht wird. Nachdem der Untersuchungsort homogen mit Infrarotstrahlung, die von der Infrarotdiode durch den Katheter via Monomodefasern in den Körper geleitet wird, ausgeleuchtet worden ist, wird das Bild der Gefäßwände mittels der Optik bzw. dem optischen System auf dem Bildsensor abgebildet. Die Strahlung tritt durch das Substrat 112 in das Photodiodenarray ein. Diese rückseitige Bestrahlung bietet wie bezugnehmend auf Fig. 3 bereits erwähnt einerseits den Vorteil, dass die optische Schnittstelle ohne Metallkontakte auskommt, und andererseits den Vorteil, dass noch eine weitere Optik zur Strahlbündelung monolithisch in das Substrat des Pixelarrays 112 integriert werden kann, nämlich in dem dem distalen Ende des Katheters zugewandten Teil des Arrays 112, der sich zwischen distalem Ende und der Oberfläche des Pixelarraysubstrats befindet, in welcher die Pixeldioden des Pixelarrays gebildet sind, so dass die Lichtempfindlichkeit jedes Pixels erhöht werden könnte, indem die integrierte Optik Strahlung auf die lichtempfindlichen Zonen der Pixeldioden, nämlich die Raumladungszonen, bündelt. Nach der Umwandlung der Photonen in Ladung/Signale werden diese über den CMOS-Chip ausgelesen, vorverarbeitet und/oder codiert und über Kabel an den externen Prozessor für die Bildverarbeitung übertragen. Die Bildverarbeitungseinheit extrahiert die gewünschte Information, bevor sie auf dem Monitor dargestellt wird. Anschließend wird diese Information in einer Patientendatenbank, wie z.B. in dem Speicher 66, gespeichert.

Es sei darauf hingewiesen, dass gemäß den im vorhergehenden beschriebenen Ausführungsbeispielen zwar immer eine Vorverarbeitungseinrichtung 50 bereits im Katheterkopf angeordnet war, dass diese Vorverarbeitung allerdings auch erst im Rahmen der Bildverarbeitung in der Bildverarbeitungseinrichtung 62 vorgenommen werden könnte. Die Vorverarbeitung bereits im Katheterkopf, wie z.B. eine Dynamikanpassung, eine Kanalanpassung, eine Herausfilterung oder eine Quellcodierung, kann dabei allerdings eventuell die Anforderungen an die Weiterleitung der Pixelinformationen bzw. Pixelmesswerte an die externe Apparatur 30 reduzieren, wie z.B. die Anzahl der notwendigen Kabel reduzieren oder dergleichen, oder die Übertragungsrate bei gleichbleibender Verkabelung erhöhen.

Wie es im vorhergehenden bereits angedeutet worden ist, ist das Anordnen weiterer Sensoren für die vorliegende Erfindung nicht wesentlich. Umgekehrt können, wie oben ebenfalls angedeutet, in dem Katheter weitere Vorrichtungen vorgesehen sein, wie z.B. solche für die Navigation des Endoskops in den Blutgefäßen. Hierzu könnte ein oder mehrere Aktuatoren vorgesehen sein, der gegebenenfalls mechanischer Natur ist, weshalb in dem Katheter auch ein mechanischer Seilzug vorgesehen sein könnte, der von dem proximalen Ende zum Katheterkopf verläuft, um diesen Aktuator ansteuern zu können.

Ferner wird darauf hingewiesen, dass sich die vorhergehenden Ausführungsbeispiele lediglich exemplarisch auf die Darstellung des kardiovaskulären Systems bezog, bzw. auf ein kardiovaskuläres Endoskop zur Darstellung der Innenwände des kardiovaskulären Systems mittels eines minimal invasiven Bildaufnahmesystems. Erfindungsgemäße Videoendoskope können jedoch ferner auch anderenorts bei der medizinischen Diagnose eingesetzt werden.

Die vorhergehenden Ausführungsbeispiele könnten als Angioskop eingesetzt werden und als diagnostisches Werkzeug den Herz- und Gefäßchirurgen bei minimal invasiv durchführbaren Herzoperationen unterstützen, wie z.B. bei der Rekonstruktion bzw. dem Ersatz von Mitral- oder Trikuspidalklappen, bei dem Verschluss eines Kammerseptumdefektes oder bei dem Einpflanzen von koronalen Bypässen. Ferner können prä-operativ verschiedene Defekte des Gefäßsystems, wie Läsionen, Anneurismen, Sklerosen und Stenosen sichtbar gemacht und bewertet werden. Intraoperativ kann die Beseitigung dieser Effekte, wie z.B. durch Einsetzen eines Stents, oder durch RF- bzw. Hochfrequenzablation oder Kryoablation, mit dem Angioskop begleitet werden. Post-operativ können diese Eingriffe sehr einfach evaluiert werden. Ein weiteres großes Anwendungsgebiet für die im vorhergehenden beschriebenen Ausführungsbeispiele ist die exakte Bewertung von Thrombosen, Embolien und Infarkten, die heutzutage in einer Gesellschaft mit zunehmend älteren Patienten eine Herausforderung darstellt. Die verbesserte Untersuchung erhöht die Sicherheit bei der nachfolgenden Therapie.

Obige Ausführungsbeispiele bilden also folglich ein diagnostisches Werkzeug und ermöglichen ein damit verbundenes Diagnoseverfahren, mit dem prä-, intra- und post-operativ Beobachtungen an bzw. in Organen und Gefäßen in realer, d.h. bluterfüllter Umgebung durchgeführt werden können. Der Arzt kann durch den Katheter, dessen distales Ende er über die Blutbahn an den Untersuchungsort heranführt, und die extrakorporale Bildverarbeitungseinheit im Monitor in das kardiovaskuläre System hineinschauen. Diese Videoendoskopievorrichtungen unterstützen den behandelnden Chirurgen bei der Navigation und Durchführung von schwierigen Eingriffen, wie z.B. am Herzen. Die obigen Ausführungen ermöglichen neuartige Diagnoseverfahren, die wiederum eine einfache morphologisch-funktionelle Bildgebung des kardiovaskulären Systems mit variablen Einsatzmöglichkeiten ermöglichen und den Arzt sowohl prä- als auch intra- als auch post-operativ begleiten. Im Gegensatz zu den Standardbildgebungsverfahren, die zur Darstellung des kardiovaskulären Systems benutzt werden, leistet dieses bildgebende Verfahren höhere Auflösung ohne ionisierende Strahlung.

## Patentansprüche

1. Videoendoskopievorrichtung (10) mit
einer Sensorvorrichtung (48); und
einem Katheter (40) zum Ausgeben von Strahlung an einem distalen Ende (69) des Katheters (40), und zum Empfangen reflektierter Strahlung an dem distalen Ende (69, 120) und Abbilden derselben auf die Sensorvorrichtung (48),
wobei die Sensorvorrichtung (48) im Katheter (40) in der Nähe des distalen Endes (69) des Katheters (40) angeordnet ist, und ausgebildet ist, um die reflektierte Strahlung in ein elektrisches Signal umzuwandeln, und wobei der Katheter (40) ausgebildet ist, um das elektrische Signal zu einem proximalen Ende (67) des Katheters (40) zu leiten, wobei die Sensorvorrichtung (48) ein Photodiodenarray (112) aufweist, das auf einer Hauptseite eines Halbleitersubstrates angeordnet ist, wobei das Photodiodenarray (112) in dem Katheter (40) achsensymmetrisch und quer zu einer Längsachse (106) des Katheters derart angeordnet ist, dass die Hauptseite des Photodiodenarrays (112) dem distalen Ende (69) abgewandt und eine dieser Hauptseite gegenüber liegende Hauptseite des Halbleitersubstrats dem distalen Ende (69) zugewandt ist.

2. Vorrichtung gemäß Anspruch 1, bei der der Katheter (40) ferner ausgebildet ist, um die Strahlung von dem proximalen Ende (67) zu dem distalen Ende (69) des Katheters (40) zu leiten.

3. Videoendoskopievorrichtung gemäß Anspruch 1, bei der die Sensorvorrichtung (48) ein Photodetektorarray (48, 112) aufweist.

4. Videoendoskopievorrichtung gemäß Anspruch 1 oder 2, bei der der Katheter (40) einen Strahlungsleiter (70) zum Leiten der Strahlung von dem proximalen Ende (67) an das distale Ende (69) aufweist.

5. Videoendoskopievorrichtung gemäß einem der vorhergehenden Ansprüche, bei der der Katheter (40) ferner eine Optik zur Aufweitung der Strahlung aufweist.

6. Videoendoskopievorrichtung gemäß einem der vorhergehenden Ansprüche, bei der die Sensorvorrichtung (48) im wesentlichen achszentral zu einer Längsachse (106) des Katheters (40) angeordnet ist, und der Strahlungsleiter (70) vom proximalen Ende (67) des Katheters (40) am Außenmantel (102) des Katheters (40) entlang, an der Sensorvorrichtung (48) vorbei, zum distalen Ende (69) des Katheters verläuft.

7. Videoendoskopievorrichtung gemäß Anspruch 6, bei der der Strahlungsleiter durch eine Mehrzahl von Monomodefasern gebildet ist, die im Querschnitt quer zur Längsachse (106) des Katheters (40) ringförmig entlang des Außenmantels (102) des Katheters (40) angeordnet sind.

8. Videoendoskopievorrichtung gemäß Anspruch 1, bei der der Katheter (40) eine Lichtquelle aufweist, die am distalen Ende (69) des Katheters (40) integriert ist, um die Strahlung am distalen Ende (69) des Katheters (40) auszugeben.

9. Videoendoskopievorrichtung gemäß einem der vorhergehenden Ansprüche, bei der der Katheter (40) eine Abbildungsoptik (46; 108a, 108b) zum Abbilden der reflektierten Strahlung auf die Sensorvorrichtung (48) aufweist.

10. Videoendoskopievorrichtung gemäß Anspruch 9 wiederum rückbezogen auf Anspruch 7, bei der die Abbildungsoptik (46) im wesentlichen achszentral zu einer Längsachse (106) des Katheters in dem Katheter (40) am distalen Ende (69) des Katheters (40) angeordnet und durch Halterungen (110) am Außenmantel (102) des Katheters (40) befestigt ist, wobei die Monomodefasern (104) durch die Halterungen (110) verlaufen.

11. Videoendoskopievorrichtung gemäß Anspruch 8, bei die Strahlungsquelle (60) eine Infrarotdiode (60) aufweist.

12. Videoendoskopievorrichtung gemäß einem der Ansprüche 1 bis 11, bei der der Katheter (40) eine Energiezuführung (80) zum Versorgen der Sensorvorrichtung (48) mit Energie aufweist.

13. Videoendoskopievorrichtung gemäß einem der Ansprüche 1 bis 12, die ferner eine Bildverarbeitungseinrichtung (62) zum Aufbereiten des elektrischen Signals aufweist, um ein Endoskopiebild zu erhalten, wobei die Bildverarbeitungseinrichtung (62) mit dem proximalen Ende (67) des Katheters (40) koppelbar ist.

14. Videoendoskopievorrichtung gemäß einem der vorhergehenden Ansprüche, bei der die Sensorvorrichtung (48) eine Vorverarbeitungselektronik (50) zur Vorverarbeitung des elektrischen Signals aufweist.

15. Videoendoskopievorrichtung gemäß Anspruch 14, bei der die Vorverarbeitungselektronik (50) derart angepasst ist, dass sie eine Dynamikanpassung, eine Kanalanpassung, eine Rauschfilterung oder eine Quellcodierung umfasst.

16. Videoendoskopievorrichtung gemäß einem der Ansprüche 1 bis 15, die ferner einen Monitor (64) aufweist, der mit der Bildverarbeitungseinrichtung (62) koppelbar ist.

17. Videoendoskopievorrichtung gemäß einem der Ansprüche 1 bis 16, die ferner ein in dem Katheter (40) angeordnetes Sensorelement (52) zum Erfassen von Druck, Temperatur oder eines ph-Wertes aufweist.

18. Videoendoskopievorrichtung gemäß einem der Ansprüche 1 bis 17, bei der die Sensorvorrichtung bei einer Betriebswellenlänge zwischen 1,5 µm und 1,8 µm oder 2,1 µm und 2,3 µm wirksam ist.

19. Videoendoskopievorrichtung gemäß einem der Ansprüche 1 bis 18, bei der die Sensorvorrichtung (48) folgendes Merkmal aufweist:
einen Chip (114) zur Signalverarbeitung, wobei der Chip (114) mit dem Photodiodenarray (112) mit Hilfe von Flip-Chip-Bonding verbunden ist, derart, dass die Hauptseite des Halbleitersubstrats, in dem das Photodiodenarray (112) gebildet ist, dem Chip (114) zugewandt ist.

20. Videoendoskopievorrichtung gemäß Anspruch 19, bei der in einer Hauptseite des Chips (114), die dem Photodiodenarray (112) zugewandt ist, eine Signalverarbeitungsschaltung zur Vorverarbeitung des elektrischen Signals integriert ist.

21. Videoendoskopievorrichtung gemäß einem der Ansprüche 1 bis 20, bei der in das Halbleitersubstrat des Photodiodenarrays (112) eine strahlungsbündelnde Optik integriert ist.

22. Videoendoskopievorrichtung gemäß einem der vorhergehenden Ansprüche, die zur kardiovaskulären Endoskopie geeignet ist.

23. Videoendoskopievorrichtung gemäß einem der vorhergehenden Ansprüche, bei der ein zusätzlicher Bildsensor vorgesehen ist, derart, dass das Bildfeld von zusätzlichem Bildsensor und Sensorvorrichtung zusammen verglichen zu einem Bildfeld der Sensorvorrichtung (48) allein vergrößert ist, oder dass dem zusätzlichen Bildsensor ein anderes Abbildungsverfahren zugrundeliegt als der Sensorvorrichtung.

## Claims

1. A video endoscopy device (10) comprising:
a sensor device (48); and
a catheter (40) for outputting radiation at a distal end (69) of the catheter (40), and for receiving reflected radiation at the distal end (69, 120), and imaging same onto the sensor device (48),
the sensor device (48) being arranged, within the catheter (40), in the vicinity of the distal end (69) of the catheter (40), and is configured to convert the radiation reflected into an electric signal, and the catheter (40) being configured to route the electric signal to a proximal end (67) of the catheter (40), the sensor device (48) comprising a photodiode array (112) arranged on a main side of a semiconductor substrate, the photodiode array (112) being arranged, within the catheter (40), to be axially symmetrical and transverse to a longitudinal axis (106) of the catheter, such that the main side of the photodiode array (112) faces away from the distal end (69), and that a main side of the semiconductor substrate which is opposite said main side faces the distal end (69).

2. The device as claimed in claim 1, wherein the catheter (40) is further configured to route the radiation from the proximal end (67) to the distal end (69) of the catheter (40).

3. The video endoscopy device as claimed in claim 1, wherein the sensor device (48) comprises a photodetector array (48, 112).

4. The video endoscopy device as claimed in claim 1 or 2, wherein the catheter (40) comprises a radiation router (70) for routing the radiation from the proximal end (67) to the distal end (69).

5. The video endoscopy device as claimed in any of the previous claims, wherein the catheter (40) further comprises optics for flaring the radiation.

6. The video endoscopy device as claimed in any of the previous claims, wherein the sensor device (48) is arranged in a manner which is essentially axially central to a longitudinal axis (106) of the catheter (40), and wherein the radiation router (70) extends from the proximal end (67) of the catheter (40) along the outer jacket (102) of the catheter (40), past the sensor device (48), and to the distal end (69) of the catheter.

7. The video endoscopy device as claimed in claim 6, wherein the radiation router is formed by a plurality of monomode fibers arranged, in cross section transverse to the longitudinal axis (106) of the catheter (40), in an annular manner along the outer jacket (102) of the catheter (40).

8. The video endoscopy device as claimed in claim 1, wherein the catheter (40) comprises a light source integrated at the distal end (69) of the catheter (40) to output the radiation at the distal end (69) of the catheter (40).

9. The video endoscopy device as claimed in any of the previous claims, wherein the catheter (40) comprises imaging optics (46; 108a, 108b) for imaging the reflected radiation onto the sensor device (48).

10. The video endoscopy device as claimed in claim 9 when referring back to claim 7, wherein the imaging optics (46) are arranged, within the catheter (40), in a manner which is essentially axially central to a longitudinal axis (106) of the catheter, at the distal end (69) of the catheter (40) and are mounted, by means of fixtures (110), to the outer jacket (102) of the catheter (40), the monomode fibers (104) extending through the fixtures (110).

11. The video endoscopy device as claimed in claim 8, wherein the radiation source (60) comprises an infrared diode (60).

12. The video endoscopy device as claimed in any of claims 1 to 11, wherein the catheter (40) comprises an energy supply (80) for supplying the sensor device (48) with energy.

13. The video endoscopy device as claimed in any of claims 1 to 12, further comprising an image processing means (62) for processing the electric signal to obtain an endoscopy image, the image processing means (62) being adapted to be coupled to the proximal end (67) of the catheter (40).

14. The video endoscopy device as claimed in any of the previous claims, wherein the sensor device (48) comprises pre-processing electronics (50) for pre-processing the electric signal.

15. The video endoscopy device as claimed in claim 14, wherein the pre-processing electronics (50) are adapted such that they include dynamics adjustment, channel adjustment, noise filtering or source encoding.

16. The video endoscopy device as claimed in any of claims 1 to 15, further comprising a monitor (64) adapted to be coupled to the image processing means (62).

17. The video endoscopy device as claimed in any of claims 1 to 16, further comprising a sensor element (52), arranged within the catheter (40), for detecting pressure, temperature or a pH value.

18. The video endoscopy device as claimed in any of claims 1 to 17, wherein the sensor device is operative at an operation wavelength of between 1.5 µm and 1.8 µm, or between 2.1 µm and 2.3 µm.

19. The video endoscopy device as claimed in any of claims 1 to 18, wherein the sensor device (48) comprises:
a chip (114) for signal processing, the chip (114) being connected to the photodiode array (112) by means of flip-chip bonding, such that the main side of the semiconductor substrate, wherein the photodiode array (112) is formed, faces the chip (114).

20. The video endoscopy device as claimed in claim 19, wherein a signal processing circuit for pre-processing the electric signal is integrated within a main side of the chip (114), said main side facing the photodiode array (112).

21. The video endoscopy device as claimed in any of claims 1 to 20, wherein beam-focusing optics are integrated into the semiconductor substrate of the photodiode array (112).

22. The video endoscopy device as claimed in any of the previous claims, suitable for cardiovascular endoscopy.

23. The video endoscopy device as claimed in any of the previous claims, wherein an additional image sensor is provided, such that the image field is enlarged by the additional image sensor and sensor device together, compared to an image field of the sensor device (48) alone, or that the additional image sensor is based on a different imaging method than the sensor device.

## Revendications

1. Dispositif de vidéoendoscopie (10), avec
un dispositif détecteur (48); et
un cathéter (40) destiné à sortir le rayonnement à une extrémité distale (69) du cathéter (40), et à recevoir le rayonnement réfléchi à l'extrémité distale (69, 120) et à reproduire celui-ci sur le dispositif détecteur (48),
le dispositif détecteur (48) étant disposé dans le cathéter (40) à proximité de l'extrémité distale (69) du cathéter (40), et étant réalisé de manière à convertir le rayonnement réfléchi en un signal électrique, et le cathéter (40) étant réalisé de manière à guider le signal électrique vers une extrémité proximale (67) du cathéter (40), le dispositif détecteur (48) présentant une rangée de photodiodes (112) qui est disposée sur une face principale d'un substrat à semi-conducteur, la rangée de photodiodes (112) étant disposée dans le cathéter (40) de manière symétrique à l'axe et transversalement à un axe longitudinal (106) du cathéter, de sorte que la face principale de la rangée de photodiodes (112) soit éloignée de l'extrémité distale (69) et qu'une face principale, opposée à cette face principale, du substrat à semi-conducteur soit orientée vers l'extrémité distale (69).

2. Dispositif selon la revendication 1, dans lequel le cathéter (40) est par ailleurs réalisé de manière à guider le rayonnement de l'extrémité proximale (67) à l'extrémité distale (69) du cathéter (40).

3. Dispositif de vidéoendoscopie selon la revendication 1, dans lequel le dispositif détecteur (48) présente une rangée de photodétecteurs (48, 112).

4. Dispositif de vidéoendoscopie selon la revendication 1 ou 2, dans lequel le cathéter (40) présente un guide de rayonnement (70) destiné à guider le rayonnement de l'extrémité proximale (67) à l'extrémité distale (69).

5. Dispositif de vidéoendoscopie selon l'une des revendications précédentes, dans lequel le cathéter (40) présente par ailleurs une optique destinée à élargir le rayonnement.

6. Dispositif de vidéoendoscopie selon l'une des revendications précédentes, dans lequel le dispositif détecteur (48) est disposé sensiblement au centre de l'axe par rapport à un axe longitudinal (106) du cathéter (40), et le guide de rayonnement (70) s'étend de l'extrémité proximale (67) du cathéters (40), le long de l'enveloppe extérieure (102) du cathéter (40), en passant par le dispositif détecteur (48), vers l'extrémité distale (69) du cathéter.

7. Dispositif de vidéoendoscopie selon la revendication 6, dans lequel le guide de rayonnement est formé par une pluralité de fibres monomode qui sont disposées à section annulaire transversale à l'axe longitudinal (106) du cathéter (40) le long de l'enveloppe extérieure (102) du cathéter (40).

8. Dispositif de vidéoendoscopie selon la revendication 1, dans lequel le cathéter (40) présente une source de lumière qui est intégrée à l'extrémité distale (69) du cathéter (40), pour sortir le rayonnement à l'extrémité distale (69) du cathéter (40).

9. Dispositif de vidéoendoscopie selon l'une des revendications précédentes, dans lequel le cathéter (40) présente une optique de reproduction (46; 108a, 108b) destinée à reproduire le rayonnement réfléchi sur le dispositif détecteur (48).

10. Dispositif de vidéoendoscopie selon la revendication 9, avec référence à la revendication 7, dans lequel l'optique de reproduction (46) est disposé sensiblement au centre de l'axe par rapport à un axe longitudinal (106) du cathéter dans le cathéter (40) à l'extrémité distale (69) du cathéter (40) et est fixée par des supports (110) à l'enveloppe extérieure (102) du cathéter (40), les fibres monomode (104) s'étendant à travers les supports (110).

11. Dispositif de vidéoendoscopie selon la revendication 8, dans lequel la source de rayonnement (60) présente une diode infrarouge (60).

12. Dispositif de vidéoendoscopie selon l'une des revendications 1 à 11, dans lequel le cathéter (40) présente une alimentation d'énergie (80) destinée à alimenter en énergie le dispositif détecteur (48).

13. Dispositif de vidéoendoscopie selon l'une des revendications 1 à 12, présentant par ailleurs un moyen de traitement d'image (62) destiné à préparer le signal électrique, pour obtenir une image d'endoscopie, le moyen de traitement d'image (62) pouvant être couplé à l'extrémité proximale (67) du cathéter (40).

14. Dispositif de vidéoendoscopie selon l'une des revendications précédentes, dans lequel le dispositif détecteur (48) présente une électronique de prétraitement (50) destinée à prétraiter le signal électrique.

15. Dispositif de vidéoendoscopie selon la revendication 14, dans lequel l'électronique de prétraitement (50) est adaptée de sorte qu'elle comporte une adaptation de dynamique, une adaptation de canal, une filtration de bruit ou un codage de source.

16. Dispositif de vidéoendoscopie selon l'une des revendications 1 à 15, présentant par ailleurs un moniteur (64) qui peut être couplé au moyen de traitement d'image (62).

17. Dispositif de vidéoendoscopie selon l'une des revendications 1 à 16, présentant par ailleurs un élément capteur (52) disposé dans le cathéter (40) et destiné à capter la pression, la température ou une valeur de pH.

18. Dispositif de vidéoendoscopie selon l'une des revendications 1 à 17, dans lequel le dispositif détecteur est actif à une longueur d'onde de fonctionnement comprise entre 1,5 µm et 1,8 µm ou entre 2,1 µm et 2,3 µm.

19. Dispositif de vidéoendoscopie selon l'une des revendications 1 à 18, dans lequel le dispositif détecteur (48) présente la caractéristique suivante:
une puce (114) destinée au traitement de signal, la puce (114) étant assemblée avec la rangée de photodiodes (112) à l'aide d'un assemblage de puce à bossage, de sorte que la face principale du substrat à semi-conducteur dans lequel est formée la rangée de photodiodes (112) soit orientée vers la puce (114).

20. Dispositif de vidéoendoscopie selon la revendication 19, dans lequel est intégré, dans une face principale de la puce (114) orientée vers la rangée de photodiodes (112), un circuit de traitement de signal destiné à prétraiter le signal électrique.

21. Dispositif de vidéoendoscopie selon l'une des revendications 1 à 20, dans lequel est intégrée, dans le substrat à semi-conducteur de la rangée de photodiodes (112), une optique regroupant le rayonnement en faisceau.

22. Dispositif de vidéoendoscopie selon l'une des revendications précédentes qui convient pour l'endoscopie cardiovasculaire.

23. Dispositif de vidéoendoscopie selon l'une des revendications précédentes, dans lequel est prévu un capteur d'image additionnel, de sorte que le champ d'image du capteur d'image additionnel et du dispositif détecteur ensemble soit agrandi, comparé à un champ d'image du dispositif détecteur (48) seul, ou que le capteur d'image additionnel se base sur un autre procédé de reproduction que le dispositif détecteur.
